# FASCICULE DE BREVET EUROPEEN

(11) **EP 4 370 907 B1**
(45) Date de publication et mention de la délivrance du brevet: **18.02.2026**
(21) Numéro de dépôt: 22743527.8
(22) Date de dépôt: 23.06.2022
(51) Int. Cl.: G01N 21/88, G01N 33/2045, G06T 7/00

(54) **PROCÉDÉ DE CONTRÔLE DES DÉFAUTS DE SURFACE D'UNE PIÈCE DE FONDERIE EN MÉTAL MONOCRISTALLIN ET SYSTÈME DE MISE EN OEUVRE**
VERFAHREN ZUR INSPEKTION VON OBERFLÄCHENDEFEKTEN AUF EINEM GUSSTEIL AUS EINKRISTALLMETALL UND SYSTEM ZUR IMPLEMENTIERUNG DAVON
METHOD FOR INSPECTING FOR SURFACE DEFECTS ON A CAST PART MADE OF SINGLE-CRYSTAL METAL AND SYSTEM FOR IMPLEMENTING SAME

(30) Priorité: 12.07.2021 FR 2107543
(43) Date de publication de la demande: 22.05.2024
(73) Titulaire: SAFRAN, 75015 Paris (FR)
(72) Inventeur: MICHAUD, Franck, 77550 Moissy-Cramayel (FR)
(74) Mandataire: Cabinet Camus Lebkiri
(86) Numéro de dépôt international: PCT/FR2022/051234
(87) Numéro de publication internationale: WO 2023/285745

(56) Documents cités:
- FR-A1- 2 988 841
- US-A1- 2021 048 384

## Description

### DOMAINE TECHNIQUE DE L'INVENTION

La présente invention concerne un procédé pour contrôler l'état de surface d'une pièce de fonderie en métal monocristallin, la surface de la pièce étant susceptible de comporter des défauts résultant d'une inhomogénéité d'orientation d'au moins une maille cristalline du métal monocristallin. L'invention concerne également un système de mise en œuvre de ce procédé de contrôle.

L'invention trouve des applications dans les domaines de la fonderie de pièces en métal ou alliage monocristallin et, en particulier, dans le domaine de la fabrication de pièces de fonderie en métal ou alliage métallique pour l'aéronautique.

### ARRIERE-PLAN TECHNOLOGIQUE DE L'INVENTION

La fonderie permet la réalisation de pièces métalliques complexes et, en particulier, des pièces aéronautiques comme les aubes de turbines moteurs haute pression. Les pièces de fonderie peuvent être fabriquées dans un métal ou un alliage monocristallin. Ces pièces en métal ou alliage monocristallin doivent alors être constituées de mailles cristallines ayant une orientation homogène. Autrement dit, l'orientation des mailles cristallines doit être identique pour toutes, ou au moins une très grande majorité, des mailles cristallines de la pièce.

En aéronautique, l'état de surface de chacune des pièces de fonderie en métal ou alliage monocristallin est contrôlé de sorte à vérifier que l'orientation cristalline est homogène sur toute la surface de la pièce. En effet, lorsque les mailles cristallines d'une pièce présentent des orientations différentes, des fissures ou autres défauts peuvent s'initier notamment à la jointure des mailles cristallines et provoquer de graves dommages sur la pièce. Pour éviter ces dommages, chaque pièce aéronautique est contrôlée et la présence de joints de grains, c'est-à-dire d'une frontière entre deux mailles cristallines d'orientations différentes, est détectée. Le niveau de non-homogénéité de l'orientation cristalline, à savoir le nombre de mailles cristallines d'orientations différentes, est déterminé. Si ce niveau dépasse un seuil d'acceptabilité prédéfini, la pièce est mise au rebus.

Une technique de référence en métallurgie pour le contrôle de l'état de surface des pièces en orientation cristalline utilise une méthode de diffraction des électrons rétrodiffusés (EBSD pour Electron Backscatter Diffraction ou BKD pour Backscatter Kikuchi Diffraction). Cette méthode met non seulement en œuvre des instruments très couteux, mais en plus elle implique une préparation importante de l'échantillon de mesure qui doit être plan et poli. De plus, la surface à analyser par balayage, ou scanning, est relativement petite (de l'ordre du mm² ou du cm²) et la mesure par scanning du faisceau d'électron est longue et n'est pas compatible avec un tri rapide de pièces de production.

D'autres méthodes optiques ont été plus récemment mises en œuvre, comme le contrôle par BRDF (Bidirectional Reflectance Distribution Fonction, en termes anglo-saxons). Ce contrôle par BRDF est une méthode utilisée par extension de la l'expérience empirique des contrôles qui étaient, auparavant, effectués à l'œil nu par des opérateurs. Cette méthode est basée sur le fait qu'après une attaque chimique de la pièce, la présence des différents grains produit visuellement une variation de contraste lumineux sur la surface de la pièce, grâce à une variation de réflectance de ladite pièce. Auparavant, un opérateur recherchait, à l'œil nu, ces variations de contraste lumineux à la surface de la pièce. Pour cela, il inclinait la pièce sous différents angles afin de voir des différences de réflectivité sur la surface de pièce. Même pour un opérateur formé et aguerri, ces opérations de contrôle étaient délicates et demandaient au contrôleur une grande concentration, car toute défaillance de l'opérateur pouvait engendrer une décision critique de sa part avec, soit l'acceptation d'une pièce dont l'état n'atteint pas le seuil d'acceptabilité, soit la mise au rebus d'une pièce acceptable. Par rapport à la méthode empirique, le contrôle par BRDF permet de faciliter le travail des opérateurs en mettant en œuvre un banc de contrôle optique afin de mesurer la fonction de réflectance de la pièce et d'en déduire la géométrie de son orientation cristalline. Un déplacement angulaire contrôlé de l'instrument d'éclairage et de mesure est nécessaire comme décrit dans les articles «Measuring crystal orientation from etched surfaces via directional reflectance microscopy » de Wang Xiaogang, dans J Mater Sci (2020) 55 :11669-11678 et «Optical characterization of grain orientation in crystalline materials » de Bernard Gaskey, dans Acta Materialia 194 (2020) 558-564).

US 2021/048384 divulgue un procédé de contrôle de l'état de surface d'une pièce de fonderie en métal monocristallin.

Les techniques de contrôle de l'état de surface des pièces basées sur la méthode de diffraction des électrons rétrodiffusés (comme l'EBSD) sont difficilement intégrables sur une chaine de production et dans le cadre de pièces mécaniques complexes. Les techniques optiques ont l'avantage de proposer plus de versatilité pour les mesures dans un environnement complexe et en contrôle non destructif mais elles sont peu adaptées au cadre industriel

Il existe donc un besoin d'un procédé et d'un dispositif de contrôle automatisé amélioré, permettant d'aider les opérateurs dans leur tâche de contrôle de l'état de surface des pièces en métal ou alliage monocristallin.

### RESUME DE L'INVENTION

Pour répondre aux problèmes évoqués ci-dessus et aider les opérateurs au contrôle de l'état de surface des pièces issue de fonderie, le demandeur propose un procédé et un système pour contrôler l'état de surface des pièces en métal ou alliage monocristallin, basés sur une analyse d'une série d'images réalisées pour différentes orientations de la polarisation de la lumière réfléchie par la surface de la pièce.

Selon un premier aspect, l'invention concerne un procédé de contrôle de l'état de surface d'une pièce de fonderie en métal monocristallin, la surface de la pièce comportant d'éventuels défauts résultant d'une inhomogénéité d'orientation d'au moins une maille cristalline du métal monocristallin, ledit procédé comportant :
- l'acquisition, par un dispositif d'acquisition d'images, d'une série d'images de la pièce de fonderie éclairée au moyen d'un dispositif d'éclairage polarisé et collimaté, puis
- l'analyse de la série d'images au moyen d'un dispositif de traitement d'image, cette analyse comportant les opérations suivantes :
   - détermination, pour chaque pixel du dispositif d'acquisition d'images, d'un vecteur d'intensité correspondant à une variation d'intensité du même pixel sur chaque image de la série d'images,
   - détermination d'une intensité moyenne de l'ensemble des pixels de chaque image de la série d'images et extraction d'une loi de variation générale de l'intensité pour la série d'images,
   - détermination d'une loi de variation individuelle de l'intensité, propre à chaque maille cristalline, et
   - détermination des pixels appartenant à une même maille cristalline,
chaque image de la série d'images étant réalisée pour un angle de polarisation différent.

L'éclairage polarisé utilisé dans ce procédé permet de rehausser le contraste de réflectance à la surface de la pièce. En outre, l'analyse de la série d'images réalisées avec des angles de polarisation différents permet de générer une image de la pièce mettant en exergue les différents grains. Ce procédé présente en outre l'avantage d'être facilement automatisable.

On comprendra que l'expression « métal monocristallin » employée dans la description et les revendications inclut tous les métaux et alliages de type monocristallin. Les pièces de fonderie à contrôler avec le procédé et/ou le système selon l'invention sont des pièces en métal monocristallin ou en alliage monocristallin dont les mailles cristallines doivent présenter une orientation homogène.

Les termes « grains » ou « mailles cristallines désorientées » ou « mailles cristallines d'orientations différentes », employés dans la description et les revendications ont une signification identique, un grain étant une maille cristalline dont l'orientation n'est pas conforme à l'orientation attendue. Un joint de grain est donc un frontière entre deux mailles cristallines d'orientations différentes.

Outre les caractéristiques qui viennent d'être évoquées dans le paragraphe précédent, le procédé de contrôle selon un aspect de l'invention peut présenter une ou plusieurs caractéristiques complémentaires parmi les suivantes, considérées individuellement ou selon toutes les combinaisons techniquement possibles :
- l'angle de polarisation est modifié par rotation d'un axe de polarisation du dispositif d'acquisition d'images.
- l'opération de détermination des pixels appartenant à une même maille cristalline est obtenue par appariement des pixels ayant des variations d'intensité proches.
- l'opération de détermination d'une loi de variation individuelle de l'intensité est obtenue par normalisation de l'intensité de chaque pixel en fonction de la loi de variation générale de l'intensité.
- le dispositif d'éclairage génère un faisceau d'une lumière incohérente, polarisée linéairement.
- le procédé comporte une opération préalable d'attaque chimique de la pièce de fonderie faisant ressortir les cristaux à la surface de la pièce de fonderie.

Un deuxième aspect de l'invention concerne un système de mise en œuvre du procédé de contrôle défini précédemment, ce système comportant :
- un dispositif d'éclairage polarisé et collimaté,
- un dispositif d'acquisition d'images, et
- un dispositif de traitement d'images.

L'association d'un dispositif d'éclairage fixe et d'une caméra fixe permet de mettre en évidence les différences de réflectance sur la surface de la pièce par simple rotation d'un polariseur devant la caméra, sans mouvement relatif de la pièce et de l'éclairage et sans problème d'ombrage ou de profondeur de champ pour les pièces courbées.

Ce système de contrôle selon un deuxième aspect de l'invention peut présenter une ou plusieurs caractéristiques complémentaires parmi les suivantes, considérées individuellement ou selon toutes les combinaisons techniquement possibles :
- le dispositif d'éclairage comporte une source lumineuse incohérente, couplée à un premier polariseur linéaire et à une optique de collimation.
- le dispositif d'acquisition d'images comporte une caméra fixe, couplée à un second polariseur mobile en rotation, chaque image de la série d'images correspondant à un réglage différent d'un axe de polarisation du second polariseur.
- le dispositif de traitement d'image comporte un dispositif d'affichage d'images affichant au moins une image de la pièce de fonderie avec un repérage des mailles cristallines désorientées.

### BREVE DESCRIPTION DES FIGURES

D'autres avantages et caractéristiques de l'invention apparaîtront à la lecture de la description qui suit, illustrée par les figures dans lesquelles :
La figure 1 représente schématiquement un exemple de système de contrôle de l'état de surface d'une pièce selon l'invention ;
La figure 2 représente, sous forme fonctionnelle, un exemple des opérations du procédé de contrôle de l'état de surface d'une pièce, selon l'invention ;
La figure 3 représente des exemples de trois images d'une série d'images réalisée pour plusieurs angles de polarisation différents ;
La figure 4 représente un exemple de la moyenne d'intensité des pixels d'une image de la série d'images, déterminée dans le procédé de la figure 2 ; et
La figure 5 représente un exemple de la variation d'intensité individuelle correspondant à trois grains différents, après analyse par le procédé de la figure 2.

### DESCRIPTION DETAILLEE

Un exemple de réalisation d'un procédé et d'un système de contrôle de l'état de surface de pièces de fonderie, configuré pour déterminer automatiquement une inhomogénéité d'orientation des mailles cristallines, est décrit en détail ci-après, en référence aux dessins annexés. Cet exemple illustre les caractéristiques et avantages de l'invention. Il est toutefois rappelé que l'invention ne se limite pas à cet exemple.

Sur les figures, les éléments identiques sont repérés par des références identiques. Pour des questions de lisibilité des figures, les échelles de taille entre éléments représentés ne sont pas respectées.

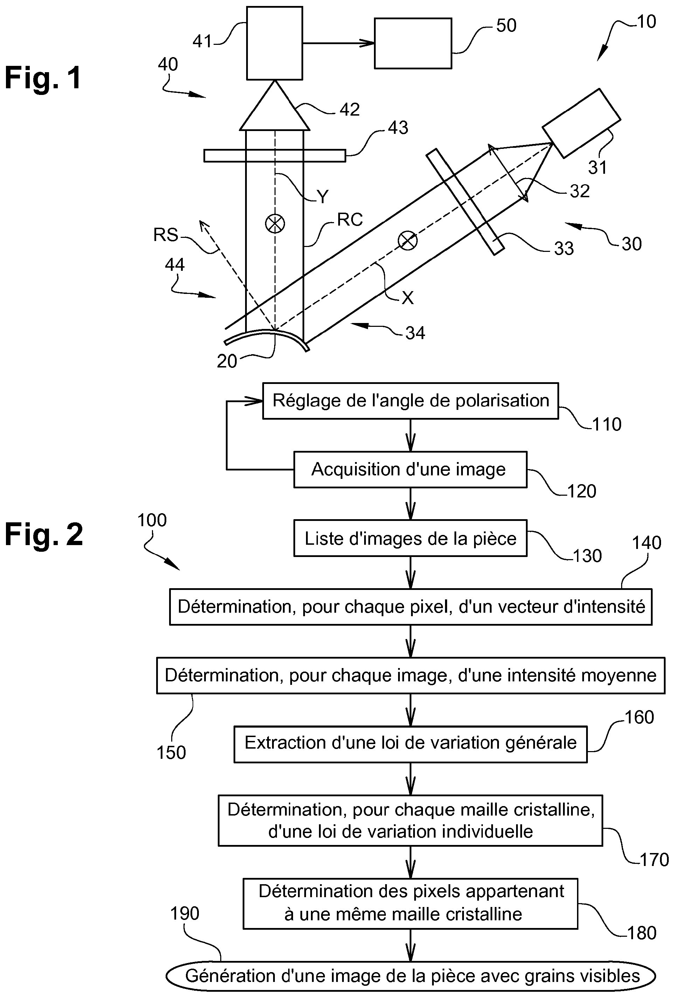

Un exemple d'un système 10 de contrôle de l'état de surface de pièces 20 en métal monocristallin issues de fonderie est représenté sur la figure 1. Ce système 10 comporte un dispositif d'éclairage 30, un dispositif d'acquisition d'images 40 et un dispositif de traitement d'image 50. Le dispositif d'éclairage 30 est conçu pour émettre une lumière incohérente (ou non-cohérente), polarisée et collimatée. En effet, le faisceau lumineux incident 34 (appelé aussi faisceau incident) doit être, d'une part, polarisé linéairement (ou transverse électrique) afin que seules les composantes du faisceau incident parallèles à l'axe de polarisation soient transmises et, d'autre part, collimaté afin que la pièce 20 reçoive un éclairage uniforme. Un éclairage uniforme de la pièce 20 permet à chaque zone de la surface de ladite pièce de recevoir la même intensité lumineuse, ce qui permet de détecter les éventuelles déviations lumineuses engendrées par des mailles cristallines désorientées, comme expliqué par la suite.

Pour cela, dans l'exemple de la figure 1, le dispositif d'éclairage 30 comporte une source lumineuse non-cohérente 31, c'est-à-dire qui ne produit pas d'interférence. L'éclairage peut être monochromatique ou polychromatique car, comme la pièce 20 est une pièce métallique, sa surface est réfléchissante sur une large bande de fréquences dans le visible. La source lumineuse 31 peut être une source lumineuse traditionnelle, une source lumineuse de type LED, une source lumineuse fibrée déportée ou non, ou toute autre source lumineuse non-cohérente.

Le dispositif d'éclairage 30 comporte également une optique de collimation 32, telle qu'une lentille de collimation, et à un polariseur linéaire 33, appelé premier polariseur, tous deux couplés à la source lumineuse 31 et alignés suivant l'axe d'éclairage X. L'ordre de positionnement de l'optique de collimation 32 et du premier polariseur 33, derrière la source lumineuse 31, a peu d'importance dès lors que le faisceau incident 34 émis par le dispositif d'éclairage 30 vers la pièce 20 est à la fois polarisé et collimaté.

Le premier polariseur 33 est un polariseur linéaire par transmission. Différentes technologies de construction d'un tel polariseur linéaire peuvent être envisagées : par exemple un film dichroïque, une micro grille ou des nanoparticules prolates. Dans l'invention, le premier polariseur 33 est choisi de préférence faiblement diffusant et avec un taux maximum d'extinction à la longueur d'onde considérée. L'axe de polarisation du premier polariseur 33, appelé axe de polarisation incidente, est fixé pour toute la durée du contrôle de la pièce 20. Le premier polariseur 33 peut donc être fixe. Selon une variante, le premier polariseur 33 peut être monté sur une monture rotative, ce qui permet de pouvoir choisir l'axe de polarisation incidente et, éventuellement, le modifier pour le contrôle d'une autre pièce 20. L'axe de polarisation incidente est, de préférence, choisi de sorte que le faisceau lumineux incident 34 soit transverse électrique, c'est-à-dire perpendiculaire au plan d'incidence. Dans l'invention, la surface réfléchissante étant une surface métallique, on considère que ladite surface est un conducteur parfait. La réflexion 44 du faisceau incident 34 se fait donc sans perte d'énergie et l'angle de réflexion obéit aux lois de Snell Descartes.

Dans le mode de réalisation représenté sur la figure 1, la source lumineuse 31 et le premier polariseur 33 sont deux éléments distincts, alignés l'un avec l'autre suivant l'axe d'éclairage X. Dans un autre mode de réalisation, la source lumineuse 31 est une source polarisée qui intègre le premier polariseur. La source lumineuse polarisée est alors, de préférence, mobile en rotation afin que l'opérateur puisse positionner ladite source lumineuse selon l'axe de polarisation incidente choisi.

Le faisceau lumineux incident 34 qui éclaire la pièce 20 est polarisé linéairement de sorte que le coefficient de réflexion de ladite pièce, appelé coefficient de réflexion de Fresnel, dépend à la fois de l'angle d'incidence et de l'état de polarisation de la lumière incidente. La réponse optique de la surface de la pièce 20, c'est-à-dire le faisceau de lumière réfléchi 44 (appelé aussi faisceau réfléchi), comporte deux composantes majeures :
- une réflexion spéculaire RS faiblement dispersive qui provient de l'état de surface moyen de la pièce 20 ( avec une rugosité aléatoire). Cette réflexion est majoritaire en intensité et garde la même orientation d'axe de polarisation que la lumière incidente.
- une réflexion RC issue des faces orientées des mailles cristallines. Cette réflexion, produite par lesdites faces orientées, peut être vue comme la réflexion commune d'une multitude de micro-miroirs orientés dans un même sens.

La réponse optique de la surface de la pièce 20 est détectée par le dispositif d'acquisition d'images 40 et analysée par le dispositif de traitement d'image 50. Le dispositif d'acquisition d'images 40 comporte une caméra 41 équipée d'un objectif 42 et couplée à un polariseur 43, appelé second polariseur, monté devant l'objectif 42 de la caméra. La caméra 41 peut être, par exemple, une caméra de type CCD ou CMOS dans le domaine du visible. L'objectif 42 de la caméra est un objectif optique dont les caractéristiques sont adaptées, en champ de vision et distance de travail, à la taille de la zone à imager de la pièce 20. Cet objectif 42 peut être, par exemple, de type télé-centrique de sorte à favoriser une grande profondeur de champ. Le second polariseur 43, aligné avec la caméra 41 et son objectif 42 suivant l'axe de réflexion Y, est de même type que le premier polariseur 33, c'est-à-dire de type linéaire. Il peut même être identique au premier polariseur.

Selon l'invention, le second polariseur 43 est mobile en rotation afin que son axe de polarisation puisse être modifié pour chaque acquisition d'image de façon à obtenir une série d'images correspondant chacune à un angle de polarisation différent. Pour cela, le second polariseur 43 peut être fixé sur une monture rotative, non représentée sur la figure, de sorte que son axe de polarisation, appelé axe de polarisation réfléchie, puisse être tourné. La monture rotative peut être manipulée manuellement par un opérateur. Elle peut, avantageusement, être motorisée de sorte à tourner automatiquement. La rotation du second polariseur 43 permet d'acquérir, au moyen de la caméra 41, plusieurs images avec des angles de polarisation différents. Si l'axe du second polariseur 43 est orienté dans le même sens que l'axe de la polarisation réfléchie, alors l'intensité lumineuse transmise dans la caméra est maximale. À l'inverse, si l'axe du second polariseur 43 est tourné de 90° (configuration dite de polariseur croisé), la transmission de l'intensité lumineuse est minimale. Entre ces deux angles, la transmission de l'intensité lumineuse suit la loi de Malus :
*Iₜᵣₐₙₛ = I_{inc}cos*²*θ,* où θ est l'angle formé entre les axes des deux polariseurs 33 et 43.

Dans un mode de réalisation, la caméra 41 est une caméra polarimétrique qui intègre directement un polariseur. Dans une telle camera polarimétrique, les pixels du capteur de la caméra sont équipés de polariseurs et sont donc sensibles à l'axe de polarisation et à l'intensité du faisceau réfléchi 44 sur le capteur. En une acquisition, il est alors possible d'obtenir les mêmes images qu'en faisant tourner l'axe de polarisation du second polariseur 43, la différence étant que le nombre d'images est limité au nombre d'angles de polarisation prédéfinis, par exemple quatre angles à 0°,45°, 90° et 135°.

Le dispositif de traitement d'image 50 est une unité de traitement, comme par exemple un ordinateur, adaptée pour réaliser les opérations de traitement d'image décrites par la suite. Il est connecté par liaison filaire ou liaison sans fil à la caméra 41 afin de recevoir la série d'images acquises par la caméra 41. Ce dispositif de traitement d'image 50 comporte au moins un dispositif d'affichage d'images, tel qu'un écran ou une imprimante, pour afficher le résultat du contrôle de la pièce 20.

Le système de contrôle 10 tel qu'il vient d'être décrit permet de mettre en œuvre une polarisation croisée entre le dispositif d'éclairage 30 et le dispositif d'acquisition d'images 40. Avec cette polarisation croisée, chaque grain correspondant à une orientation cristalline particulière présente, après réflexion sur la surface de la pièce 20 et transmission dans le second polariseur 43, une réponse optique particulière en intensité qui dépend de la position choisie de l'axe de polarisation incidente et du réglage de l'axe de polarisation réfléchie. En effet, il est admis que les facettes de chaque maille cristalline orientent différemment l'axe de polarisation de la lumière réfléchie, comme cela est décrit dans l'article intitulé « Correlation of Polarized Light Phenomena With the Orientation of Some Metal Crystals », de C. J. Newton et H. C. Vacher, dans Journal of Research of the National Bureau of Standards Vol. 53, No. 1, Juillet 1954. Autrement dit, chaque désorientation de maille cristalline propage le faisceau lumineux dans la caméra 41 avec un coefficient de réflexion qui lui est propre, car dépendant de la direction de polarisation du faisceau incident. Ce principe permet de distinguer les différents grains composant la surface de la pièce 20.

La réponse optique des différents grains de la surface de la pièce 20 est analysée au moyen des étapes 140 à 180 du procédé de contrôle de l'état de surface de la pièce 20. Un exemple de ce procédé de contrôle 100 est représenté de façon fonctionnelle sur la figure 2. Comme on le comprend de ce qui précède, les premières opérations du procédé de contrôle 100 consistent en la génération d'une série 130 d'images de la surface de la pièce 20 ou de la zone de la pièce 20 à contrôler. En effet, en fonction des dimensions de la pièce 20 à contrôler, le dispositif d'acquisition d'images 40 peut réaliser des images de toute la surface de la pièce 20 ou d'une zone de cette surface. Par exemple, pour une aube de turbine, dont les dimensions sont relativement grandes, le contrôle de l'état de surface de l'aube peut être réalisé zone par zone, chaque zone faisant l'objet d'une série d'images analysées comme expliqué par la suite, chaque série d'images étant analysée les unes à la suite des autres. Dans la suite de la description, le procédé de contrôle 100 sera décrit pour des images de toute la surface de la pièce, étant entendu que les images peuvent concerner uniquement une zone de la surface de la pièce.

La série 130 d'images de la surface de la pièce 20, appelées plus simplement images de la pièce, est réalisée au moyen du dispositif d'acquisition d'images 40 en réalisant une image de la pièce pour chaque angle de polarisation et en modifiant l'angle de polarisation avant chaque prise d'image. On appelle « angle de polarisation » l'angle entre l'axe de polarisation de premier polariseur 33 et l'axe de polarisation du second polariseur 43. Autrement dit, avant chaque prise d'image, le second polariseur 43 est réglé sur une position différente de sa position précédente afin de modifier l'axe de polarisation d'analyse et, par conséquent, l'angle de polarisation. Ainsi, le procédé de contrôle 100 comporte une première opération 110 de réglage de l'angle de polarisation, par rotation de l'axe de polarisation du second polariseur 43. Il comporte ensuite une opération 120 d'acquisition, par la caméra 41, d'une image de la pièce 20 pour l'angle de polarisation réglé. Plusieurs images sont ainsi réalisées. En rebouclant l'opération 120 d'acquisition d'images et l'opération 110 de réglage de l'angle de polarisation, on obtient une série 130 d'images de la pièce. Cette série 130 d'images de la pièce 20, constituée de plusieurs images de la même pièce 20 sous plusieurs angles de polarisation, est ensuite analysée au moyen des opérations 140 à 180 du procédé 100 afin de déterminer la présence de grains, c'est-à-dire de mailles cristallines ayant des orientations différentes de celle de la plupart des mailles cristallines de la surface de la pièce 20. La série d'images 130 comporte plusieurs images, par exemple, une ou plusieurs dizaines d'images, le nombre d'images devant être suffisant pour permettre la construction d'une courbe sensiblement sinusoïdale, comme expliqué par la suite. Dans un exemple pratique, la rotation de l'axe de polarisation du second polariseur 43 peut être modifiée d'un pas de 10° dans un intervalle compris entre 0° et 360°. Il est à noter que l'acquisition des images pour les angles de polarisation compris entre 0° et 180° sont en théorie identiques à celles pour les angles compris entre 180° et 360° du fait de la périodicité de rotation de l'axe du second polariseur 43 ; l'acquisition d'images pour des angles de polarisation compris entre 0° et 180° peut donc suffire ; cependant il peut être choisi d'acquérir également des images sur l'intervalle compris entre 180° et 360°, par exemple pour augmenter le rapport signal à bruit des données déterminées dans la suite du procédé 100 et/ou si l'axe de polarisation incidente n'est pas parfaitement orienté perpendiculairement au plan d'incidence.

Pour faire ressortir les cristaux à la surface de la pièce 20 et ainsi faciliter l'analyse, le procédé de contrôle de l'invention peut comporter une opération préliminaire, non représentée sur les figures, d'attaque chimique de la surface de la pièce 20. En effet, l'attaque chimique a pour effet de creuser légèrement la matière à la surface de la pièce 20 afin de faire apparaitre à fleur de surface les mailles cristallines. Cette attaque chimique est effectuée avant la réalisation de la série d'images (opérations 110 à 130).

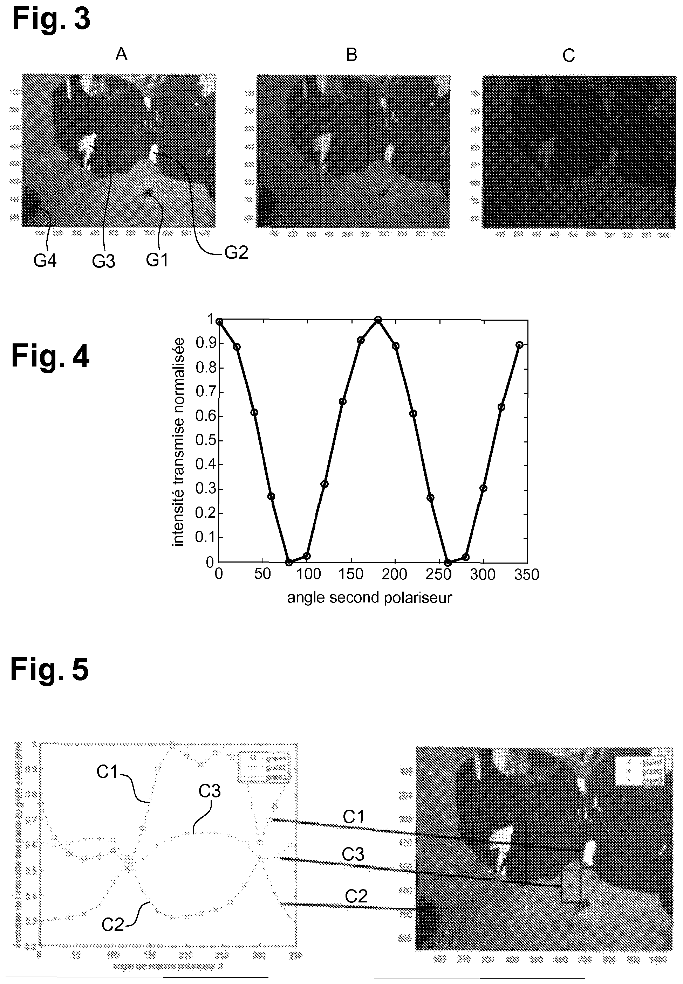

Un exemple d'une série de trois images est représenté sur la figure 3. Ces trois images représentent la même portion d'aube de turbine, après attaque chimique, pour trois positions de l'axe de polarisation du second polariseur 43 et donc trois angles de polarisation différents. L'image A correspond à la portion d'aube pour un angle de polarisation de 0° ; l'image B montre la même portion d'aube pour un angle de polarisation de 45° ; l'image C montre toujours la même portion d'aube pour un angle de polarisation de 90°. Il ressort de ces trois images A, B et C qu'il existe plusieurs portions de la pièce 20 où les orientations cristallines diffèrent. Ces portions de la pièce 20, référencées G1, G2, G3 et G4, correspondent à des défauts de surface de la pièce 20.

Après réalisation de la série d'images 130, le procédé de contrôle 100 comporte des opérations pour analyser cette série d'images. Cette analyse comporte tout d'abord une opération 140 de détermination d'un vecteur d'intensité, pour chaque pixel du capteur de la caméra 41. Puisque toutes les images de la série d'images 130 correspondent à la même zone de la pièce 20 et que la pièce 20 n'est pas déplacée, les images de la série d'images 130 peuvent se superposer les unes aux autres ; il est alors possible de regarder l'évolution, c'est-à-dire la variation, des niveaux de gris d'un même pixel à travers la série d'images. En effet, un même pixel présente un niveau de gris différent sur chaque image de la série d'images 130. La variation de ce niveau de gris du pixel à travers les différentes images de la série d'images 130 forme un vecteur d'intensité dudit pixel.

Le procédé 100 comporte ensuite une opération 150 de détermination d'une intensité moyenne normalisée de l'ensemble des pixels de chaque image de la série d'images 130. Cette opération 150 consiste à calculer la moyenne de l'intensité lumineuse de tous les pixels d'une même image et à en déduire, à l'opération 160, une loi de variation générale pour la série d'images 130. Cette loi de variation générale correspond à la loi dite « de Malus » qui est la loi relative à la transmission, à travers le second polariseur 43, du faisceau lumineux 44 réfléchi par la pièce 20 et correspondant à la rugosité moyenne de la surface de ladite pièce 20. L'intensité issue des faces orientées des mailles cristallines est majoritaire dans le signal total car la pièce 20 n'est pas un réseau cristallin pur. La figure 4 représente, sous la forme d'une courbe globalement sinusoïdale, un exemple de la loi de variation générale obtenue à la fin de l'opération 160. Cette courbe de la figure 4 montre un exemple d'intensité moyenne normalisée, transmise par le second polariseur 43, pour plusieurs angles de polarisation compris entre 0° et 360°, le maximum d'intensité étant obtenu lorsque le premier et le second polariseurs sont en phase, le minimum d'intensité étant obtenu lorsque le premier et le second polariseurs sont croisés.

Le procédé 100 comporte ensuite une opération 170 de détermination d'une loi de variation individuelle de l'intensité, c'est-à-dire une loi de variation propre à chaque maille cristalline. Pour cela, l'opération 170 consiste, pour chaque image de la série d'images, à normaliser l'intensité de chaque pixel de l'image entre 0 et 1, en divisant cette intensité par la valeur de l'intensité moyenne de l'image correspondante. Cela revient à diviser le vecteur d'intensité de chaque pixel de la série d'images par la loi de variation générale, ou loi de malus. Cette opération 170 permet d'éliminer la composante de variation de la réflexion spéculaire de la surface de la pièce 20 et de rehausser les variations d'intensité dues aux réflexions sur les facettes des mailles cristallines. En effet, comme expliqué précédemment, il est admis que les facettes d'une maille cristalline orientent différemment l'axe de polarisation de la lumière réfléchie de sorte que chaque grain correspondant à une orientation particulière de la maille cristalline présente une variation d'intensité spécifique en fonction de la position de l'axe de rotation du second polariseur 43. Ainsi, à l'issue de l'opération 170, la loi de variation propre à chaque maille cristalline (appelée loi de variation individuelle), est déterminée.

Un exemple des variations d'intensité de pixels appartenant à trois grains différents est représenté sur la figure 5, ces variations étant déterminées au moyen des différentes opérations du procédé de contrôle 100 décrites précédemment. En particulier, la courbe C1 correspond à la variation d'intensité des pixels d'un premier grain, la courbe C2 correspond à la variation d'intensité des pixels d'un deuxième grain et la courbe C3 correspond à la variation d'intensité des pixels d'un troisième grain. Ces trois courbes de variations, très différentes les unes des autres, montrent que les mailles cristallines correspondant à ces courbes ont des orientations différentes. Si l'orientation de ces mailles cristallines était homogène, les trois courbes seraient sensiblement parallèles. Il est à noter que ces différences de variations d'intensité sont notables du fait de la normalisation de l'intensité des pixels ; c'est l'opération de normalisation qui rend perceptible les variations d'intensité engendrées par la désorientation des mailles cristallines.

Le procédé de contrôle 100 comporte ensuite une opération 180 de détermination des pixels appartenant à une même maille cristalline. Cette opération 180 consiste à appairer les pixels ayant des variations d'intensité proches. En effet, des pixels présentant des variations d'intensité proches, c'est-à-dire similaires ou fortement corrélées, appartiennent à un même grain ou présentent une même orientation cristalline. L'appariement des pixels est réalisé en balayant tous les pixels de la série d'images et en vérifiant, par une méthode de similarité, si les vecteurs d'intensité de deux pixels présentent des similarités. Si la mesure de similarité entre les deux vecteurs d'intensité est supérieure à un seuil prédéterminé, alors les deux pixels sont considérés comme appariés. Au contraire, si la mesure de similarité entre les deux vecteurs d'intensité est inférieure au seuil prédéterminé, alors les deux pixels sont considérés comme n'appartenant pas à une même maille cristalline. Plusieurs méthodes de similarité peuvent être utilisées comme, par exemple, la corrélation, la méthode dite de « squared intensity differences », la méthode dite d'« absolute intensity differences » ou celle appelée « mean absolute difference ». La segmentation des différents grains, sur l'image, est ensuite déduite de cette opération d'appariement.

Le procédé de contrôle 100 comporte finalement une opération 190 de génération d'une image de la pièce 20 sur laquelle les mailles cristallines désorientées sont repérées, par exemple par un contour de la maille, un encadré, etc. Un exemple d'une telle image est représenté sur la figure 5 où les trois grains, ou mailles cristallines désorientées, sont entourés d'un cadre rectangulaire.

Le procédé de contrôle 100 peut être couplé à une technique classique de mesure multidirectionnelle de la réflectance, telle que décrite notamment dans les articles « Measuring crystal orientation from etched surfaces via directional reflectance microscopy » de Wang Xiaogang, dans J Mater Sci (2020) 55 :11669-11678 et «Optical characterization of grain orientation in crystalline materials » de Bernard Gaskey, dans Acta Materialia 194 (2020) 558-564. Le couplage du procédé de l'invention avec cette technique classique permettrait de minimiser le nombre d'angles d'éclairage et améliorer les rapport signal à bruit sur les faibles signaux.

Bien que décrit à travers un certain nombre d'exemples, variantes et modes de réalisation, le procédé selon l'invention pour contrôler l'état de surface d'une pièce de fonderie et le système de mise en œuvre de ce procédé comprennent divers variantes, modifications et perfectionnements qui apparaîtront de façon évidente à l'homme du métier, étant entendu que ces variantes, modifications et perfectionnements font partie de la portée de l'invention.

## Revendications

1. Procédé de contrôle (100) de l'état de surface d'une pièce de fonderie (20) en métal monocristallin, la surface de la pièce comportant d'éventuels défauts (G1, G2, G3, G4) résultant d'une inhomogénéité d'orientation d'au moins une maille cristalline du métal monocristallin, ledit procédé comportant :
- l'acquisition (110, 120), par un dispositif d'acquisition d'images (40), d'une série d'images (130) de la pièce de fonderie éclairée au moyen d'un dispositif d'éclairage (30) polarisé et collimaté, puis
- l'analyse (140-180) de la série d'images (130) au moyen d'un dispositif de traitement d'image (50), cette analyse comportant les opérations suivantes :
o détermination (140), pour chaque pixel du dispositif d'acquisition d'images, d'un vecteur d'intensité correspondant à une variation d'intensité du même pixel sur chaque image de la série d'images,
o détermination (150) d'une intensité moyenne de l'ensemble des pixels de chaque image de la série d'images et extraction (160) d'une loi de variation générale de l'intensité pour la série d'images,
o détermination (170) d'une loi de variation individuelle de l'intensité, propre à chaque maille cristalline, et
o détermination (180) des pixels appartenant à une même maille cristalline,
chaque image de la série d'images (130) étant réalisée pour un angle de polarisation différent, l'angle de polarisation étant l'angle entre un axe de polarisation d'un premier polariseur (33) faisant partie du dispositif d'éclairage (30) et un axe de polarisation d'un second polariseur (43) faisant partie du dispositif d'acquisition d'images (40).

2. Procédé de contrôle selon la revendication 1, **caractérisé en ce que** l'angle de polarisation est modifié par rotation d'un axe de polarisation du dispositif d'acquisition d'images (40).

3. Procédé de contrôle selon la revendication 1 ou 2, **caractérisé en ce que** l'opération (180) de détermination des pixels appartenant à une même maille cristalline est obtenue par appariement des pixels ayant des variations d'intensité proches.

4. Procédé de contrôle selon l'une des revendications 1 à 3, **caractérisé en ce que** l'opération (170) de détermination d'une loi de variation individuelle de l'intensité est obtenue par normalisation de l'intensité de chaque pixel en fonction de la loi de variation générale de l'intensité.

5. Procédé de contrôle selon l'une quelconque des revendications 1 à 4, **caractérisé en ce que** le dispositif d'éclairage (30) génère un faisceau d'une lumière incohérente, polarisée linéairement.

6. Procédé de contrôle selon l'une quelconque des revendications 1 à 5, **caractérisé en ce qu'**il comporte une opération préalable d'attaque chimique de la pièce de fonderie faisant ressortir les cristaux à la surface de la pièce de fonderie.

7. Système de mise en œuvre du procédé de contrôle selon l'une quelconque des revendications 1 à 6, **caractérisé en ce qu'**il comporte :
- un dispositif d'éclairage (30) polarisé et collimaté,
- un dispositif d'acquisition d'images (40), et
- un dispositif de traitement d'images (50).

8. Système de contrôle selon la revendication 7, **caractérisé en ce que** le dispositif d'éclairage (30) comporte une source lumineuse incohérente (31), couplée à un premier polariseur linéaire (33) et à une optique de collimation (32).

9. Système de contrôle selon la revendication 7 ou 8, **caractérisé en ce que** le dispositif d'acquisition d'images (40) comporte une caméra fixe (41, 42), couplée à un second polariseur (43) mobile en rotation, chaque image de la série d'images (130) correspondant à un réglage différent d'un axe de polarisation du second polariseur (43).

10. Système de contrôle selon l'une quelconque des revendications 7 à 9, **caractérisé en ce que** le dispositif de traitement d'image (50) comporte un dispositif d'affichage d'images affichant au moins une image de la pièce de fonderie (20) avec un repérage des mailles cristallines désorientées.

## Patentansprüche

1. Verfahren (100) zur Kontrolle des Oberflächenzustands eines Gussteils (20) aus einkristallinem Metall, wobei die Oberfläche des Teils mögliche Fehler (G1, G2, G3, G4) aufweisen kann, die aus einer ungleichen Ausrichtung mindestens eines Kristallgitters des einkristallinen Metalls resultieren, wobei das Verfahren umfasst:
- das Erfassen (110, 120) einer Bilderserie (130) des Gussstücks, das mittels einer polarisierten und kollimierten Beleuchtungseinrichtung (30) beleuchtet wird, durch eine Bilderfassungseinrichtung (40), dann
- die Analyse (140-180) der Bilderserie (130) mittels einer Bildverarbeitungsvorrichtung (50), wobei diese Analyse die folgenden Schritte umfasst:
o Bestimmung (140) für jedes Pixel der Bildaufnahmevorrichtung eines Intensitätsvektors, der einer Intensitätsänderung desselben Pixels auf jedem Bild der Bilderserie entspricht,
o Bestimmung (150) einer durchschnittlichen Intensität aller Pixel jedes Bildes der Bilderserie und Extraktion (160) eines allgemeinen Intensitätsänderungsgesetzes für die Bilderserie,
o Bestimmung (170) eines individuellen Intensitätsänderungsgesetzes, das für jedes Kristallgitter spezifisch ist, und
o Bestimmung (180) der Pixel, die zu demselben Kristallgitter gehören,
wobei jedes Bild der Bilderserie (130) für einen anderen Polarisationswinkel aufgenommen wird, wobei der Polarisationswinkel der Winkel zwischen einer Polarisationsachse eines ersten Polarisators (33), der Teil der Beleuchtungsvorrichtung (30) ist, und einer Polarisationsachse eines zweiten Polarisators (43), der Teil der Bildaufnahmevorrichtung (40) ist, ist.

2. Kontrollverfahren gemäß Anspruch 1, **dadurch gekennzeichnet, dass** der Polarisationswinkel durch Drehen einer Polarisationsachse der Bildaufnahmevorrichtung (40) verändert wird.

3. Kontrollverfahren gemäß Anspruch 1 oder 2, **dadurch gekennzeichnet, dass** der Vorgang (180) der Bestimmung der Pixel, die zu demselben Kristallgitter gehören, durch Abgleichen der Pixel mit ähnlichen Intensitätsänderungen erreicht wird.

4. Kontrollverfahren gemäß einem der Ansprüche 1 bis 3, **dadurch gekennzeichnet, dass** der Vorgang (170) der Bestimmung eines individuellen Intensitätsänderungsgesetzes durch Normalisierung der Intensität jedes Pixels in Abhängigkeit vom allgemeinen Intensitätsänderungsgesetz erfolgt.

5. Kontrollverfahren gemäß einem der Ansprüche 1 bis 4, **dadurch gekennzeichnet, dass** die Beleuchtungseinrichtung (30) einen Strahl aus inkohärentem, linear polarisiertem Licht erzeugt.

6. Kontrollverfahren gemäß einem der Ansprüche 1 bis 5, **dadurch gekennzeichnet, dass** es einen vorherigen chemischen Ätzvorgang des Gussteils umfasst, durch den die Kristalle an der Oberfläche des Gussteils zum Vorschein kommen.

7. System zur Durchführung des Kontrollverfahrens gemäß einem der Ansprüche 1 bis 6, **dadurch gekennzeichnet, dass** es umfasst:
- eine polarisierte und kollimierte Beleuchtungseinrichtung (30),
- eine Bildaufnahmevorrichtung (40) und
- eine Bildverarbeitungsvorrichtung (50).

8. Kontrollsystem gemäß Anspruch 7, **dadurch gekennzeichnet, dass** die Beleuchtungseinrichtung (30) eine inkohärente Lichtquelle (31) umfasst, die mit einem ersten linearen Polarisator (33) und einer Kollimationsoptik (32) gekoppelt ist.

9. Kontrollsystem gemäß Anspruch 7 oder 8, **dadurch gekennzeichnet, dass** die Bildaufnahmevorrichtung (40) eine feststehende Kamera (41, 42) umfasst, die mit einem zweiten, drehbaren Polarisator (43) gekoppelt ist, wobei jedes Bild der Bilderserie (130) einer unterschiedlichen Einstellung einer Polarisationsachse des zweiten Polarisators (43) entspricht.

10. Kontrollsystem gemäß einem der Ansprüche 7 bis 9, **dadurch gekennzeichnet, dass** die Bildverarbeitungsvorrichtung (50) eine Bildanzeigevorrichtung umfasst, die mindestens ein Bild des Gussteils (20) mit einer Markierung der desorientierten Kristallgitter anzeigt.

## Claims

1. A method (100) for inspecting the surface condition of a single-crystal metal foundry piece (20), the surface of the piece including possible defects (G1, G2, G3, G4) resulting from an inhomogeneous orientation of at least one crystal lattice of the single-crystal metal, said method including:
- acquiring (110, 120), by an image acquisition device (40), a series of images (130) of the foundry piece lit by means of a polarised, collimated lighting device (30), and then
- analysing (140-180) the series of images (130) by means of an image processing device (50), this analysis including the following operations:
∘ determining (140), for each pixel of the image acquisition device, an intensity vector corresponding to an intensity variation of the same pixel in each image in the series of images,
∘ determining (150) an average intensity of all the pixels of each image in the series of images and extracting (160) a law of general intensity variation for the series of images,
∘ determining (170) a law of individual intensity variation, specific to each crystal lattice, and
∘ determining (180) the pixels belonging to a same crystal lattice,
each image in the series of images (130) being made for a different polarisation angle, the polarisation angle being the angle between an axis of polarisation of a first polariser (33) forming part of the lighting device (30) and an axis of polarisation of a second polariser (43) forming part of the image acquisition device (40).

2. The inspection method according to claim 1, **characterised in that** the polarisation angle is modified by rotating an axis of polarisation of the image acquisition device (40).

3. The inspection method according to claim 1 or 2, **characterised in that** the operation (180) of determining the pixels belonging to a same crystal lattice is obtained by matching the pixels having alike intensity variations.

4. The inspection method according to one of claims 1 to 3, **characterised in that** the operation (170) of determining a law of individual intensity variation is obtained by normalising the intensity of each pixel as a function of the law of general intensity variation.

5. The inspection method according to any of claims 1 to 4, **characterised in that** the lighting device (30) generates a beam of incoherent, linearly polarised light.

6. The inspection method according to any of claims 1 to 5, **characterised in that** it includes a prior operation of chemically etching the foundry piece revealing the crystals on the surface of the foundry piece.

7. A system for implementing the inspection method according to any of claims 1 to 6, **characterised in that** it includes:
- a polarised, collimated lighting device (30),
- an image acquisition device (40), and
- an image processing device (50).

8. The inspection system according to claim 7, **characterised in that** the lighting device (30) includes an incoherent light source (31) coupled to a first linear polariser (33) and to collimation optics (32).

9. The inspection system according to claim 7 or 8, **characterised in that** the image acquisition device (40) includes a fixed camera (41, 42) coupled to a second rotationally movable polariser (43), each image in the series of images (130) corresponding to a different setting of an axis of polarisation of the second polariser (43).

10. The inspection system according to any of claims 7 to 9, **characterised in that** the image processing device (50) includes an image display device displaying at least one image of the foundry piece (20) with marking of the disoriented crystal lattices.
